# EUROPEAN PATENT APPLICATION

(11) **EP 3 508 171 A1**
(43) Date of publication of application: **10.07.2019**
(21) Application number: 18214244.8
(22) Date of filing: 19.12.2018
(51) Int. Cl.: A61F 2/00

(54) **PACKAGE INCLUDING AN AREAL MEDICAL DEVICE**

(30) Priority: 05.01.2018 DE 102018100195
(71) Applicant: Johnson & Johnson Medical GmbH, 22851 Norderstedt (DE)
(72) Inventor: Griffiths, Denise, 22417 Hambrurg (DE); Heni, Yannick, 78532 Tuttlingen (DE)
(74) Representative: Uexküll & Stolberg

(57) **Abstract**

A package, which includes an areal medical device like a surgical mesh, comprises a folder (1) supporting the areal medical device. The folder (1) comprises a base panel (10), which has two side edges (12, 13) opposing each other and which includes at least one tab (14, 15, 16, 17) adapted for holding the areal medical device on the base panel (10). The folder (1) further comprises two side flaps (20, 21), each of the side flaps (20, 21) emerging from a respective one of said side edges (12, 13) of the base panel (10) and being folded about said respective side edge (12, 13) onto the base panel (10) into a respective folded state. The side flaps (20, 21) and the at least one tab (14, 15, 16, 17) cover at most 15% of the area of the areal medical device.

## Description

The invention relates to a package including an areal medical device, as for example a flexible surgical mesh implant, e.g. for the treatment of hernia.

US 2012/0217176 A1 discloses a package including a surgical mesh, which is supported on a base panel of an inner component by means of several tabs. An outer component forms an outer layer of the base panel as well as a front panel, which is folded onto the surgical mesh and secured by means of two side flaps made of both the inner component and the outer component, which are mechanically interlocked.

A flap card folder comprising a base panel including several cuts forming tabs for holding an areal medical device, a front panel having a window and being hingedly connected to the base panel so that it can be folded onto the areal medical device, as well as a locking flap is known from US D719443 S.

The sterilization of dyed surgical meshes, e.g. by ethylene oxide, often results in bleaching including a discoloration, in particular when paper or cardboard of a supporting package can take up dye constituents from the surgical mesh. To largely avoid this effect or render it inconspicuous, a folder like that disclosed in US D719443 S can be opened during sterilization so that the surgical mesh is not or only slightly covered. Such a procedure, however, is laborious, impedes the processing of the packages and increases costs.

The object of the present invention is the provision of a package including an areal medical device, which can be handled in a convenient manner, in particular during sterilization and in use.

This object is achieved by the package including an areal medical device, as defined in claim 1. Advantageous versions of the invention follow from the dependent claims.

The package according to the invention includes an areal medical device and comprises a folder supporting the areal medical device. The term "areal" means that the medical device is essentially two-dimensional or flat, with its dimension in a direction perpendicular to the plane in which its area extends being small compared to a typical dimension of its area. Examples for areal medical devices are flexible surgical mesh implants, e.g. a hernia mesh or a film-laminated mesh implant, and other kinds of areal medical devices are conceivable as well. Such areal medical devices are well known in the art.

The folder comprises a base panel, which has two side edges opposing each other and which includes at least one tab adapted for holding the areal medical device on the base panel. In advantageous versions, the folder includes more than one tab, e.g. two, three, four or even more tabs. The folder further comprises two side flaps, each of the side flaps emerging from a respective one of said side edges of the base panel and being folded about said respective side edge onto the base panel (and onto the areal medical device held on the base panel) into a respective folded state. In the folded state, the side flaps and the at least one tab cover at most 15% of the area of the areal medical device.

The folder serves as a generally flat primary package for the areal medical device. This primary package, i.e. the folder including the areal medical device, can be accommodated in a sealed pouch, as well known in the art. The material of the pouch may be a laminate including an aluminum film, which is impervious to water and oxygen. Once the primary package has been sterilized and the pouch has been sealed, the primary package stays sterile within the pouch. Advantageous versions of the invention extend to the folder including the areal medical device plus the sealed pouch.

The folder proves to be particularly advantageous in a sterilization process with ethylene oxide gas. During sterilization, the areal medical device is held on the base panel by means of the at least one tab, which does not cover much of the area of the areal medical device. The side flaps of the folder are folded onto the base panel and the areal medical device placed thereon. This primary package is put into a pouch, while at least one edge of the pouch (its mouth) is not yet sealed. In this state, the interior of the pouch is exposed to ethylene oxide gas. The side flaps are somewhat elastic to keep the mouth of the pouch open. This effect (spacing feature) is facilitated because the side flaps are generally relatively small (covering less than 15% of the area of the medical device), which involves relatively short lever arms of the side flaps (measured from the side edges of the base panel serving as fold lines of the flaps) so that the pouch cannot keep the side flaps tightly closed. Thus, the gas gets easy access to the medical device. Since the side flaps and the at least one tab cover not more than 15% of the area of the medical device, its top face (i.e. the face of the areal medical device pointing away from the base panel of the folder) is essentially not in contact with the material of the folder. Thus, any dye constituents in the top region of the medical device essentially cannot migrate from the medical device to the material of the folder, and the top face of the areal medical device does not show much discoloration like bleaching or fading of color. At the end of the sterilization process, vacuum can be applied, and the mouth of the pouch is sealed. The sterilization process, which is generally known in the art, may be performed on existing equipment.

The spacing feature provided by the side flaps, as explained in the preceding paragraph, has another advantage: It prevents the package from being too flat and avoids sharp edges and corners so that it is improbable that the seal of the sterile barrier pouch is penetrated during transit.

The package according to the invention has further advantages. When removed from the pouch, the areal medical device is held on the folder, which provides some protection, e.g. the folder prevents the medical device from drops within the operation theatre. Because of the relatively small size of the side flaps, a large part of the medical device is visible, even when the flaps are closed, so that the shape, orientation and size of the medical device are visible on first sight. Moreover, the medical device can be easily grasped and removed from the folder.

In advantageous versions of the invention, the side flaps, when folded, are fixed to the base panel. To this end, each of the side flaps may include at least one nose inserted through a corresponding slit in the base panel when the respective side flap is in its folded state. Such a nose can be provided in different designs. For example, it may be formed as an extension to a side flap, or it may be shaped as a nose tab formed by a cut in a side flap. The fixation of the side flaps increases the rigidity of the folder, but does not deteriorate the spacing feature explained above.

By the positions of the slits, it is possible to adjust a desired degree of the spacing feature, utilizing the elastic properties of the side flaps. For example, when a side flap includes two nose-like extensions, in the folded state the side flap will form a higher arch if the slits are closer to the fold line of this side flap, thus increasing the spacing feature.

The base panel may include a plurality of tabs arranged for holding areal medical devices of different shapes and/or sizes. Generally, a few tabs are sufficient to hold a given medical device safely; even one tab might be sufficient. If the base panel includes additional tabs, they can be used to hold alternatively a medical device having a different shape or a different size. In this way, one type of folder can be designed for several different types of areal medical devices, which is economic and saves cost.

The at least one tab may be formed by a cut in the base panel, e.g. by an arcuate cut, which avoids sharp corners and permits insertion of an edge region of an areal medical device without damaging the device. Correspondingly, a plurality of tabs can be formed by a plurality of cuts in the base panel.

In advantageous versions of the invention, the folder is made in one piece. That means, it just comprises one component. Preferably, the folder does not include panels and/or flaps in addition to the base panel and the two side flaps already explained. And preferably, the folder does not include glue for attaching the side flaps to the base panel. Such a folder can be easily and quickly loaded with an areal medical device. It can be designed for rather large medical devices, e.g. for a size of 30 cm × 50 cm. In the prior art, packages for surgical meshes of this size consist of two components (see US 2012/0217176 A1), or they use glue. Thus, the package according to the invention is economic.

The folder may be made of cardboard or paper, e.g. of biocompatible paper having an areal weight of 220 g/cm². Cardboard and paper are able to accommodate some humidity, which might diffuse through on outer pouch or the seals thereof on the long term, so that a moisture-sensitive medical device stored in the folder is protected.

The base panel may have a central axis, and both side flaps may have the same size and may be symmetrically arranged with respect to the central axis. Both side flaps, when in the folded state, may touch the areal medical device.

The thickness of the folder including the areal medical device in the folded state of the side flaps may be in the range of from 1 mm to 4 mm, measured perpendicularly to the base panel, preferably in the range of from 2 mm to 3 mm. A thickness or height of 2 mm to 3 mm, e.g., is large enough for allowing a vacuum drying process or a sterilization with ethylene oxide, as explained above, but small enough to ensure a sufficient rigidity of the package and to avoid substantial shrinkage.

It has already been explained in the context of sterilization that the folder including the areal medical device can be accommodated in a sealed pouch. The folder may be the only packaging component within the pouch.

In the following, the invention and its advantages are summarized once more.

The folder of the package according to the invention is a flat card-like dispensing package for areal medical devices, e.g. for hernia repair based on a film structure. It is particularly suitable for a fully dyed medical device, which tends to fade out during sterilization, but it can be used for all kinds of areal medical devices, in particular flat mesh-based devices. The folder is a primary package which fixates the medical device during transit and prevents it from drops within the operation theatre.

The package according to the invention solves two main problems:
First, it avoids or minimizes the fading or bleaching of the medical device, which is caused by sterilization and also by drying processes. To prevent this effect, the areal medical device is secured on the card-like folder essentially without being covered. Comparable packages of the prior art (US D719443 S) have a flap card design and have to be opened which, however, is much more laborious.
Second, the package is compatible with existing manufacturing processes and performs well during existing drying and sterilization processes. To allow drying and sterilization, including the application of vacuum, a kind of spacing feature is inherent to the folder to provide a minimum volume within an essentially flat sterile barrier system (pouch) in order to allow gas exchange.

In the package according to the invention, the folder covers as little as possible of the areal medical device while implementing a spacer that provides a slightly three-dimensional shape to it. Thus, both of the above main problems are solved. Moreover, the folder can be made of qualified materials that are already established for medical device folder packages which are accommodated in flat pouches as a sterile barrier system and which keep an areal medical device like a mesh flat to avoid unallowable bends. The package according to the invention can be easily and quickly loaded with the medical device. And due to the fact that no self-adhesive label is required, much material is saved and the packaging process is accelerated, compared to prior art paper folders for surgical meshes. During storage, manufacturing or distribution, areal medical devices of different sizes and shapes can be held on the back panel of one size of folder. Since at least 85% of the medical device is visible to the end user, irrespective of the actual size of the medical device, the user is able to immediately identify the shape, orientation and size of the medical device. Additionally, an easy and free access and a sterile dispense of the medical device is possible, without pulling it out of a tightly folded package. Since at least 85% of the areal medical device are not covered, a flexible device can be easily removed from the folder with the side flaps remaining in the folded state, even when the side flaps cover part of the margin of the medical device.

In the following, the invention is further explained by means of embodiments. The drawings show in
- Figure 1: a top view onto a first embodiment of a folder of a package according to the invention in an unfolded state,
- Figure 2: a top view onto the folder of Figure 1 in the unfolded state holding a first type of surgical mesh,
- Figure 3: a top view onto the folder of Figure 1 in the unfolded state holding a second type of surgical mesh,
- Figure 4: a three-dimensional view of the folder of Figure 1 in the unfolded state,
- Figure 5: a three-dimensional view of the folder of Figure 1 in the folded state,
- Figure 6: a top view onto a second embodiment of a folder of a package according to the invention in the unfolded state,
- Figure 7: a top view onto a third embodiment of a folder of a package according to the invention in the unfolded state,
- Figure 8: a three-dimensional view of the folder of Figure 7 in the folded state, and
- Figure 9: a top view onto a forth embodiment of a folder of a package according to the invention in the unfolded state.

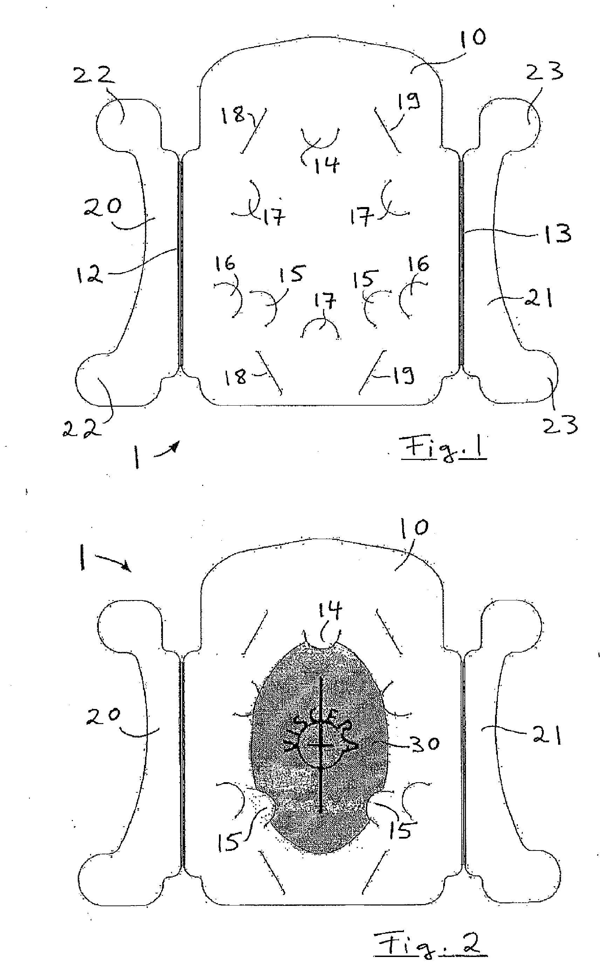

Figure 1 illustrates the folder of a first embodiment of a package, which also includes an areal medical device and a sealed pouch, in which the folder holding the areal medical device can be accommodated. Since areal medical devices like surgical meshes and sealed pouches are well known in the art, these components of the package are not considered in detail, and the following description focuses on several embodiments of the folder and its handling.

The top view in Figure 1 shows a first embodiment of the folder, which is designated by 1. The folder 1 comprises a base panel 10 which has two opposing side edges 12 and 13. The area of the base panel 10 is provided with a plurality of tabs 14, 15, 16 and 17, which are formed by an arcuate cut in the material of the base panel 10 each. Moreover, the base panel 10 is provided with two slits 18 and two slits 19 formed as cuts in the material of the base panel 10.

The folder 1 further comprises two side flaps 20 and 21, which emerge from the side edges 12 and 13, respectively. In the embodiment, the base panel 10 and the side flaps 20, 21 are made in one piece, e.g. punched from biocompatible paper material having an areal weight of, e.g., 220 g/cm². The side edges 12 and 13 of the base panel 10 are designed as fold lines (crest lines) for the side flaps 20 and 21, respectively. In the embodiment, these fold lines each comprise a plurality of parallel indentations in the material of the folder 1 so that each fold line has a certain width and provides a smooth fold. The side flap 20 includes two noses 22, and the side flap 21 includes two noses 23. In the embodiment, the noses 22 and 23 are formed as extensions to the respective side flaps 20 and 21.

Figure 2 illustrates the folder 1 in the unfolded state (as in Figure 1) holding a first type of a surgical mesh 30, which is a flexible areal medical device. In Figure 2, the surgical mesh 30 is held on the base panel 10 by three of the plurality of tabs, i.e. by the tab 14 and both tabs 15. The surgical mesh 30 is flexible and can be easily attached on the base panel 10 by slightly lifting the tabs 14 and 15 and shifting margin areas of the surgical mesh 30 under the respective tabs 14, 15. In the embodiment, the surgical mesh 30 is provided with marks to facilitate its handling (see, e.g., DE 10 2015 013 989 A1). These marks are easily visible when the surgical mesh 30 is presented on the folder 1.

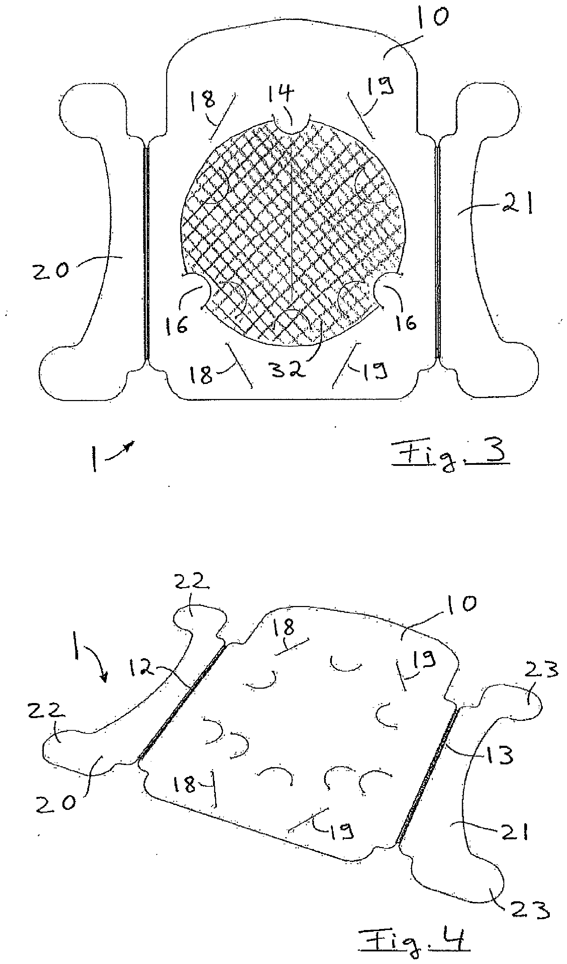

Figure 3 shows the folder 1 of Figure 1 holding a circular surgical mesh 32, the shape of which is different from that of the elliptical surgical mesh 30. In Figure 3, the tab 14 and both tabs 16 are used to hold the surgical mesh 32. This demonstrates that different types of areal medical devices can be held on one type of folder 1, when the tabs on the base panel 10 are arranged in an appropriate manner. In Figures 2 and 3, the tabs 14, 15 and 16 are used. Figure 1 shows additional tabs, i.e. tabs 17, which can be utilized for holding other types or sizes of surgical meshes.

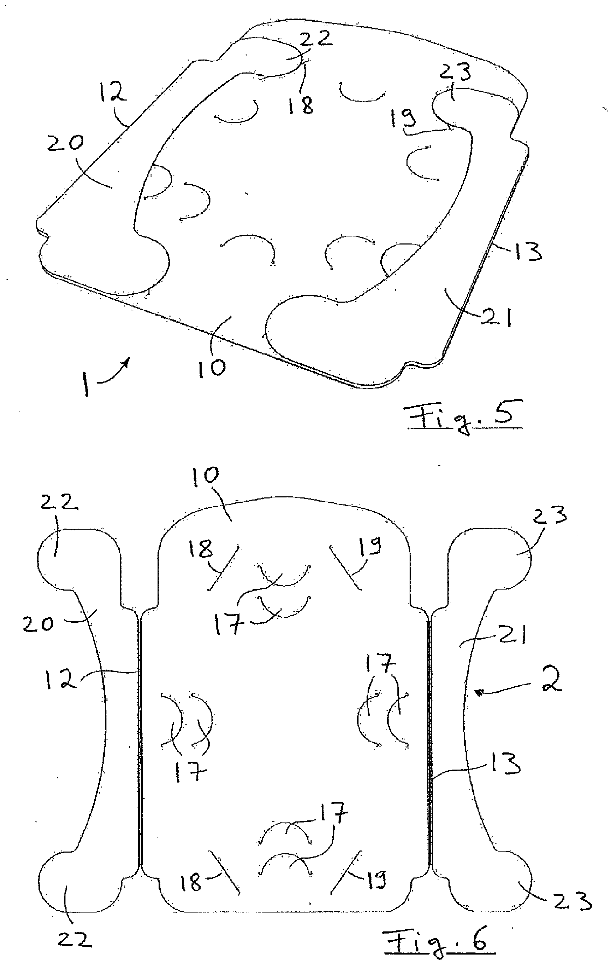

Figure 4 illustrates the folder 1 of Figure 1 in a three-dimensional view, in the unfolded state as in Figure 1. The folded state, in which the side flap 20 is folded about the side edge or fold line 12 onto the base panel 10 and the side flap 21 is folded about the side edge or fold line 13 onto the base panel 10, is shown in Figure 5.

Figure 5 is for illustration purposes and does not display the areal medical device. When using the folder 1, first an areal medical device like the elliptical surgical mesh 30 or the circular surgical mesh 32 is attached to the base panel 10, as shown in Figures 2 and 3, and afterwards the side flaps 20 and 21 are folded onto the base panel 10 and onto the medical device. In the next step (which is not indicated in Figure 5), the noses 22 and 23 are put through the respective slits 18 and 19 in order to fix the side flaps 20 and 21 in their folded state. Preferably, first the lower noses are pushed through the respective slits, and afterwards the upper noses, according to the representation in Figure 5. These steps can be performed quickly, and any adhesive or adhesive labels are not required.

In the folded state, the side flaps 20, 21 as well as any tabs 14, 15, 16, 17 involved in holding the medical device cover at most 15% of the area of the medical device. Therefore, the elliptical surgical mesh 30 is not covered at all by the side flaps 20, 21, and the circular surgical mesh 32 is just slightly covered at part of its margin. In both cases, the respective surgical mesh 30, 32, which is flexible, can be easily taken from the folder 1, while the side flaps 20, 21 stay attached to the base panel 10 by means of the noses 22, 23 and slits 18, 19.

The exact position of the slits 18 and 19 determines an amount of upwardly directed vaulting of the side flaps 20 and 21. The closer the slits 18, 19 are located to the respective side edges 12, 13, the more pronounced is the vaulting effect of the side flaps 20, 21. This vaulting effect should not be so strong that the side flaps 20, 21 kink. If a vaulting effect is not desired, the distance of the slits 18, 19 to the respective side edges 12, 13 should be sufficiently large. In any case, when the folder 1 is in the folded state, as shown in Figure 5, it exhibits at least a slight spacer effect, in particular when the fold lines at the side edges 12, 13 of the base panel 10 are relatively wide. The spacer effect is useful during sterilization of the folder and the medical device held therein, see below.

Figure 6 shows a second embodiment of a folder, which is designated by 2. The folder 2 is similar to folder 1, for which reason the same reference numerals are used in Figures 6 and 1. The folder 2 is larger than folder 1, and its side flaps 20 and 21 as well as the arrangement of the tabs (here all designated by 17) are more symmetric than in Figure 1.

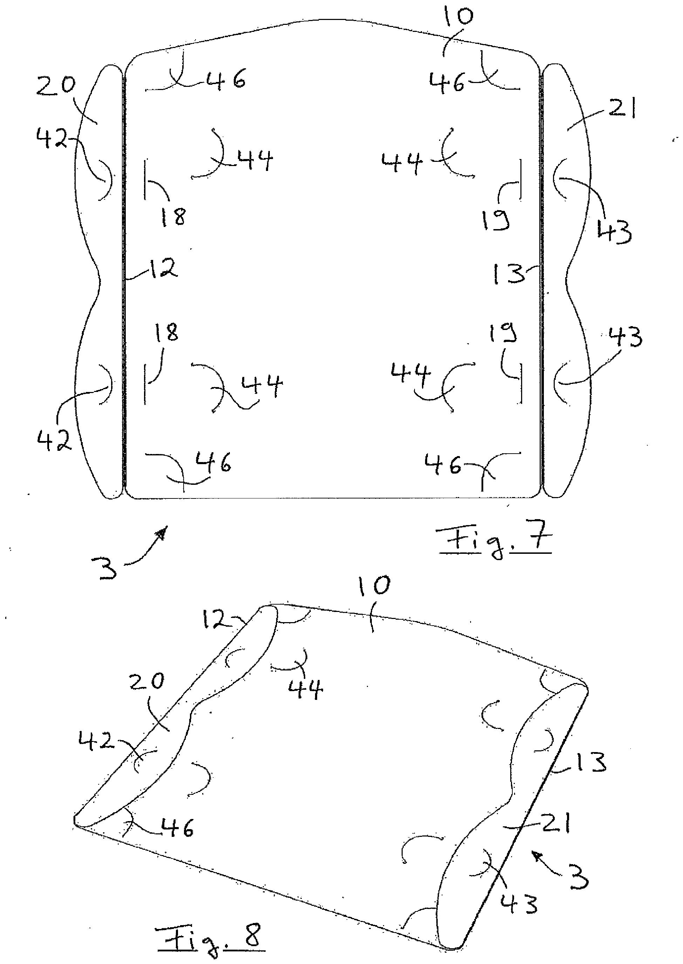

Figure 7 illustrates a third embodiment of a folder, which is designated by 3. Again, the same reference numerals are used for parts corresponding to each other.

The main difference between folder 3 and the folders 1 and 2 is the design of the noses put into slits 18 and 19 to keep the side flaps 20 and 21 in the respective folded state. In the folder 3, these noses are designed as nose tabs 42, 43 formed by adequate cuts in the side flaps 20, 21.

The base panel of folder 3 includes four tabs 44 for holding a smaller surgical mesh and four tabs 46 for holding a large essentially rectangular surgical mesh. The design of the folder 3 is particularly suited for larger surgical meshes, wherein the side flaps 20, 21 just cover narrow margins of the respective surgical mesh when in their folded states.

The folded state of folder 3 is shown in Figure 8, wherein the nose tabs 42, 43 have not yet been put through the respective slits 18, 19 on the base panel 10.

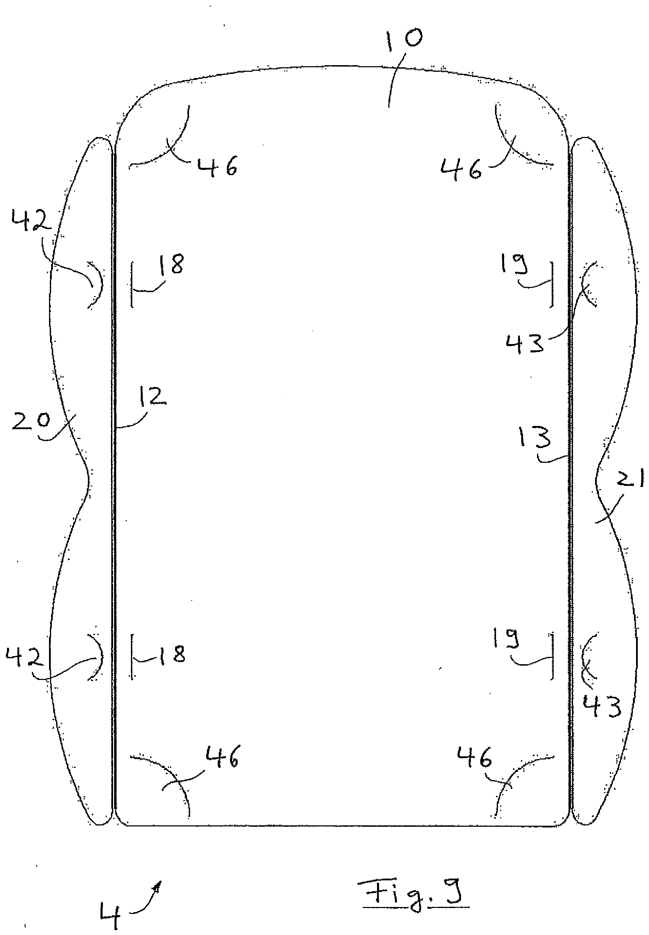

Figure 9 illustrates a folder 4 of a fourth embodiment, which is designed for a very large rectangular surgical mesh held by four tabs 46 on the base panel 10. The general design of the folder 4 is the same as that of folder 3, and again the same reference numerals are used for parts corresponding to each other.

So far, it has already been described (in particular in the context of folder 1) how an areal medical device can be placed on the base panel 10 of a folder, how the side flaps 20, 21 are folded and attached to the base panel 10, and how the medical device can be removed from the folder. Folders 2, 3, and 4 are handled in the same or in an analogous way, compared to folder 1.

It has already been mentioned that usually a package does not only comprise an areal medical device held in a folder, but also a sealed pouch. The sealed pouch encloses the folder including the medical device to protect it and keep it sterile. Such a pouch may be made of laminated film material including an aluminum foil and may have a flat rectangular shape. Initially, three edges of the pouch may be closed, and the folder holding the medical device can be inserted into the pouch via its forth edge.

To sterilize the folder and the medical device, ethylene oxide gas can be used. In this case, the gas enters the pouch via the fourth edge. Because of the spacer effect provided by the folder, as explained above, the gas can easily reach the medical device inside the pouch. Moreover, as explained as well, the top face of the medical device is largely not in contact with the folder, which avoids discoloration or fading of the medical device. After removal of the gas, e.g. by means of a vacuum pump, optionally followed by one or more than one vacuum drying step, the pouch is sealed along its fourth edge, under sterile conditions.

## Claims

1. Package including an areal medical device, the package comprising a folder (1; 2; 3; 4) supporting the areal medical device (30; 32), wherein
the folder (1; 2; 3; 4) comprises a base panel (10), which has two side edges (12, 13) opposing each other and which includes at least one tab (14, 15, 16, 17; 44, 46) adapted for holding the areal medical device (30; 32) on the base panel (10),
the folder (1; 2; 3; 4) further comprises two side flaps (20, 21), each of the side flaps (20, 21) emerging from a respective one of said side edges (12, 13) of the base panel (10) and being folded about said respective side edge (12, 13) onto the base panel (10) into a respective folded state, and
the side flaps (20, 21) and the at least one tab (14, 15, 16, 17) cover at most 15% of the area of the areal medical device (30; 32).

2. Package according to claim 1, wherein each of the side flaps (20, 21) includes at least one nose (22, 23; 42, 43) inserted through a corresponding slit (18, 19) in the base panel (10) when the respective side flap (20, 21) is in its folded state.

3. Package according to claim 2, wherein at least one nose (22, 23) is formed as an extension to a side flap (20, 21) .

4. Package according to claim 2, wherein at least one nose is shaped as a nose tab (42, 43) formed by a cut in a side flap (20, 21).

5. Package according to any one of claims 1 to 4, wherein the base panel (10) includes a plurality of tabs (14, 15, 16, 17; 44, 46) arranged for holding areal medical devices (30; 32) of different shapes and/or sizes.

6. Package according to any one of claims 1 to 5, wherein at least one tab (14, 15, 16, 17; 44, 46) is formed by a cut in the base panel (10).

7. Package according to any one of claims 1 to 6, wherein the folder (1; 2; 3; 4) is made in one piece.

8. Package according to any one of claims 1 to 7, wherein the folder (1; 2; 3; 4) does not include panels and/or flaps in addition to said base panel (10) and said two side flaps (20, 21).

9. Package according to any one of claims 1 to 8, wherein the folder (1; 2; 3; 4) is made of cardboard or paper.

10. Package according to any one of claims 1 to 9, wherein the base panel (10) has a central axis and both side flaps (20, 21) have the same size and are symmetrically arranged with respect to the central axis.

11. Package according to any one of claims 1 to 10, wherein both side flaps (20, 21), when in the folded state, touch the areal medical device (32).

12. Package according to any one of claims 1 to 11, wherein the thickness of the folder (1; 2; 3; 4) including the areal medical device (30; 32) in the folded state of the side flaps (20, 21) is in the range of from 1 mm to 4 mm, measured perpendicularly to the base panel (10), preferably in the range of from 2 mm to 3 mm.

13. Package according to any one of claims 1 to 12, wherein the folder (1; 2; 3; 4) including the areal medical device (30; 32) is accommodated in a sealed pouch.

14. Package according to claim 13, wherein the folder (1; 2; 3; 4) is the only packaging component within the pouch.

15. Package according to any one of claims 1 to 14, wherein the areal medical device comprises a surgical mesh implant (30; 32).
